(19) Europäisches Patentamt
European Patent Office
Office européen des brevets

(11) Publication number: 0 468 119 A1

# (12) EUROPEAN PATENT APPLICATION

(21) Application number: 90402129.2

(51) Int. Cl.[5]: C07F 9/6561, A61K 31/675

(22) Date of filing: 24.07.90

(43) Date of publication of application:
29.01.92 Bulletin 92/05

(84) Designated Contracting States:
FR

(71) Applicant: MERRELL DOW PHARMACEUTICALS INC.
2110 East Galbraith Road
Cincinnati Ohio 45215-6300(US)

(72) Inventor: Halazy, Serge
2 Rue Hans Haug, Wolfisheim
F-67200 Strasbourg(FR)

(74) Representative: Gillard, Marie-Louise et al
Cabinet Beau de Loménie 55, Rue d'Amsterdam
F-75008 Paris(FR)

## (54) Novel carbocyclic analogs of certain nucleosides.

(57) This invention relates to novel carbocyclic analogs of certain nucleosides, to the process for their preparation and to their use as anti-viral agents.

EP 0 468 119 A1

This invention relates to novel carbocyclic analogs of certain nucleosides, to the process for their preparation and to their use as anti-viral agents.

## BACKGROUND

AIDS is an immunosuppressive or immunodestructive disease that predisposes subjects to fatal opportunistic infections. Characteristically, AIDS is associated with a progressive depletion of T-cells, especially the helper-inducer subset bearing the $OKT_4$ surface marker.

Human immunodeficiency virus (HIV) has been reproducibly isolated from patients with AIDS or with the symptoms that frequently precede AIDS. HIV is cytopathic and appears to preferentially infect and destroy T-cells bearing the $OKT_4$ marker, and it is now generally recognized that HIV is the etiological agent of AIDS.

Since the discovery that HIV is the etiological agent of AIDS, numerous proposals have been made for anti-HIV chemotherapeutic agents that may be effective in treating AIDS sufferers. Thus, for example, European Patent Specification No. 196 1 85 describes 3'-azido-3'-deoxythymidine (which has the approved name zidovudine), its pharmaceutically acceptable derivatives and their use in the treatment of human retrovirus infections including AIDS and associated clinical conditions. Vince et al., Antiviral Research, 9 - (1/2), 120 (1988) describes certain carbocyclic purine nucleosides (in particular (±)-9-(cis-4-(hydroxymethyl)-2-cyclopentenyl) guanine and their use against HIV.

World wide, hepatitis B virus (HBV) is a viral pathogen of major consequence. It is most common in Asian countries, and prevalent in sub-Saharan Africa. The virus is aetiologically associated with primary hepatocellular carcinoma and is thought to cause 80% of the world's liver cancer. In the United States more than ten thousand people are hospitalized for HBV illness each year, an average of 250 die with fulminant disease.

The United States currently contains an estimated pool of 500,000 - 1 million infectious carriers. Chronic active hepatitis will develop in over 25% of carriers, and often progresses to cirrhosis. It is estimated that 5,000 people die from HBV-related cirrhosis each year in the USA, and that perhaps 1,000 die from HBV-related liver cancer. Even when a universal HBV vaccine is in place, the need for effective anti-HBV compounds will continue. The large reservoir of persistently infected carriers, estimated at 220 million worldwide, will receive no benefit from vaccination and will continue at high risk for HBV induced liver disease. This carrier population serves as the source of infection of susceptible individuals perpetuating the instance of disease particularly in endemic areas or high risk groups such as intravenous drug abusers and homosexuals. Thus, there is a great need for effective anti-viral agents, both to control the chronic infection and reduce progression to hepatocellular carcinoma.

Clinical effects of infection with the HBV virus range from headache, fever, malaise, nausea, vomiting, anorexia and abdominal pains. Replication of the virus is usually controlled by the immune response, with a course of recovery lasting weeks or months in humans, but infection may be more severe leading to persistent chronic liver disease as outlined above. In "Viral infections of Humans" (second edition, Ed., Evans, A.S. (1982) Plenum Publishing Corporation, New York), Chapter 12 describes in some detail, the aetiology of hepatitis infections.

Hepatitis B virus (HBV) is a small DNA containing virus which infects humans. It is a member of the class of closely related viruses known as the hepadnaviruses, each member of which selectively infects either mammalian or avian hosts, such as the woodchuck and the duck. Recent insights into the mechanism of replication of the hepadnavirus genome indicate the importance of reverse transcription of an RNA intermediate, suggesting that the reverse transcriptase is a logical chemotherapeutic target.

It has now been discovered that carbocyclic analogs of certain purine nucleosides as referred to below, are useful for the treatment or prophylaxis of viral infections such as for example hepatitis B and retroviral infections, especially AIDS.

More specifically this invention relates to compounds of the formula

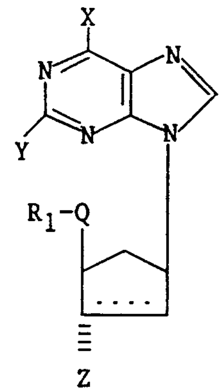

the optical and geometric isomers and mixtures thereof, and the pharmaceutically acceptable salts thereof, wherein the dotted line represents a facultative double bond,

X is $OR_2$, NHR, Cl, or $SCH_3$ with $R_2$ being a $C_{1-6}$ lower alkyl, H, or $C_{3-6}$ cycloalkyl,

Y is H or $NH_2$,

Z is H, F or $N_3$, with the proviso that when the dotted line represents a double bond Z can only be H,

$R_1$-Q is-$OCH_2P(O)(OR)_2$,-$CH_2OCH_2P(O)(OR)_2$ or-$CH_2C(A)(A)P(O)(OR)_2$, each A independently be H, F or Cl, and each R independently be H or $C_{1-6}$ alkyl.

The term $C_{1-6}$ alkyl includes the straight and branched chain hydrocarbyl radicals including methyl, ethyl, propyl, isopropyl, butyl, t-butyl, pentyl and hexyl. The term Q-$R_1$, as expressed in formula I is defined as -$OCH_2P(O)(OR\ )_2$, -$CH_2OCH_2P(O)(OR)_2$ or $CH_2C(A)(A)P(O)(OR)_2$ in order to avoid any ambiguity as to the bonding of the Q moiety to the $R_1$ and cyclopentyl moieties. However, throughout this specification, for the convenience of better understanding, the $R_1$ and Q moieties, when the context dictates, will be used and defined separately,$R_1$ being

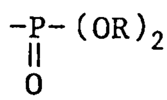

and Q being $OCH_2$-,

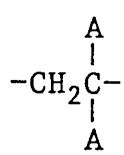

and -$CH_2OCH_2$-.

The expression "pharmaceutically acceptable acid addition salts" is intended to apply to any non-toxic organic or inorganic acid addition salts of the base compounds of formula 1. Illustrative inorganic acids which form suitable salts include hydrochloric, hydrobromic, sulfuric, and phosphoric acids and acid metal salts such as sodium monohydrogen orthophosphate and potassium hydrogen sulfate. Illustrative organic acids which form suitable salts include the mono-, di-, and tricarboxylic acids. Illustrative of such acids are, for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, and 2-phenoxybenzoic acids. Other organic acids which form suitable salts are the sulfonic acids such as methane sulfonic acid and 2-hydroxyethane sulfonic acid. Either the mono- or the di-acid salts can be formed, and such salts can exist in either a hydrated or a substantially anhydrous form. The acid salts are prepared by standard techniques such as by dissolving the free base in an aqueous or aqueous-alcohol solution or other suitable solvent containing the appropriate acid and isolating by evaporating the solution, or by reacting the free base in an organic solvent in which case the salt separates directly or can be obtained by concentration of the solution. In general the acid addition salts of the compounds of this invention are

crystalline materials which are soluble in water and various hydrophilic forms, demonstrate higher melting points and an increased stability.

The optical and geometrical isomers of the compounds of formula I may best be prepared by processes designed to enrich the production of the geometrical isomers and/or the optical isomers. However, mixtures may be resolved or isolated according to conventional and standard procedures well known in the art, e.g. chromatographic separation, fractional crystallization, use of optically active acids, enzymatic resolution and the like.

The preparation of the compounds of this invention may be effected by standard chemical processes and techniques analogously known and well understood by those of ordinary skill in the art. In general the route synthesis for any individual compound will depend on the specific substituents of each molecule and upon the ready availability of intermediates necessary; again such factors being appreciated by those of ordinary skill in the art.

To prepare compounds of this invention which are embraced by the sub-formula

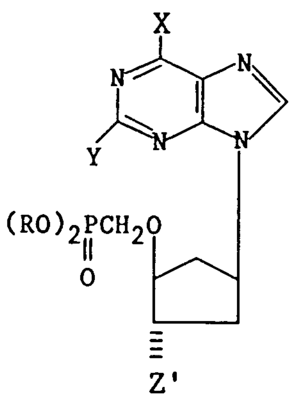

Ia

wherein X, Y, and R are as previously defined and Z' is H or F, procedures illustrated by the following reaction scheme may be employed. (For convenience,the $-OCH_2P(O)(OR')_2$ moiety wherein R' is ethyl may also be represented by $R_1'Q_1$ wherein $R_1'$ represents the $-P(O)(OEt)_2$ moiety and $Q_1$ represents the "$-OCH_2$" moiety.)

REACTION SCHEME A

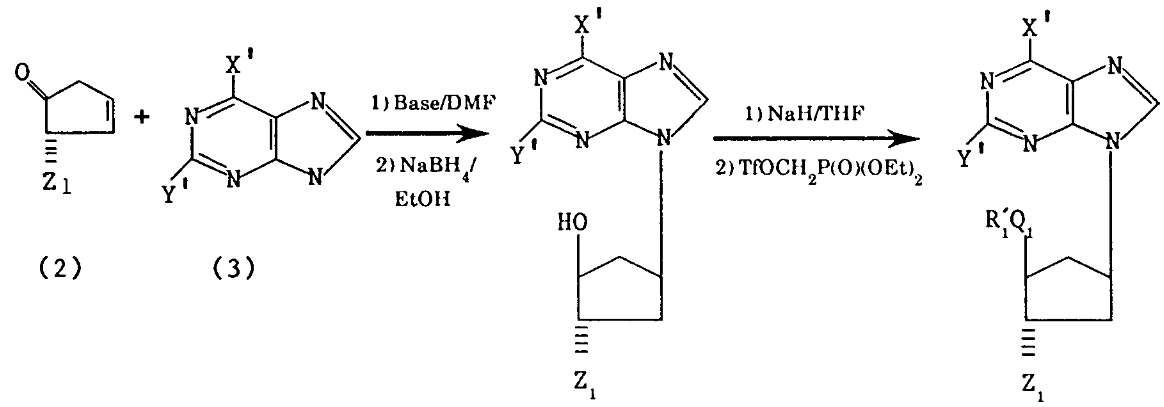


(2) (3) (4) (5)

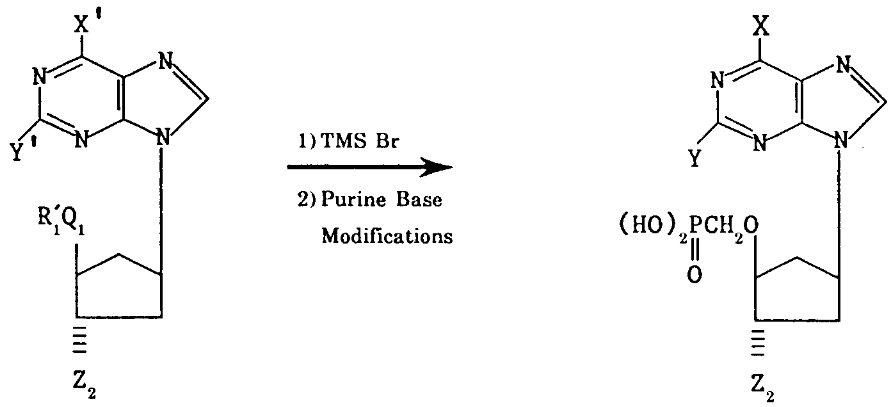


(5a) (Ia)

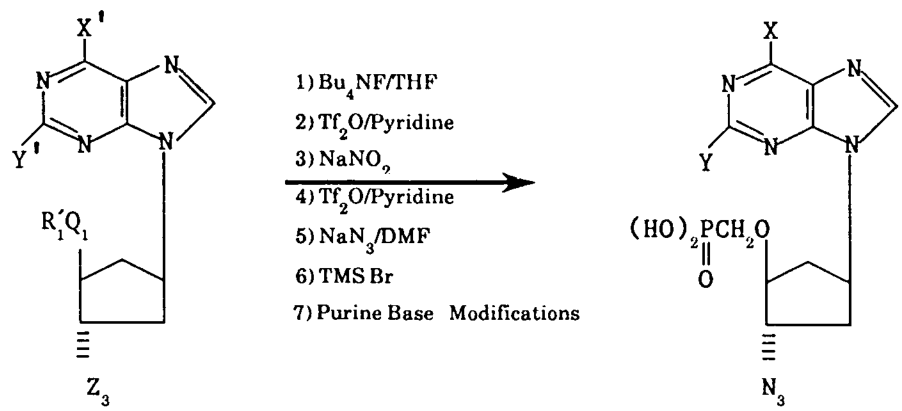


(5b) (Ib)

wherein X and Y are as previously defined, X' is Cl, Y' is H or Cl, $Z_1$ is H, F or $OSi(CH_3)_2$(t-bu), $Z_2$ is H or F, and $Z_3$ is $OSi(CH_3)_2$(t-bu).

The condensation of the 6-chloro purine (3) with the cyclopenten-one (2) is effected by reaction in the presence of catalytic amounts (usually 1-2%) of a base such as 1,8-diazabicyclo [5.4.0] undec-7-ene (DBU), KOH, $K_2CO_3$ or other suitable base in a non-reactive solvent, preferably dimethylformamide (DMF) at about -20 °C under an inert atmosphere (e.g. argon) to obtain a keto-derivative which is reduced to its corresponding alcohol by treatment with sodium borohydride in ethanol (EtOH) at about -15° to -30 °C. The resulting alcohol is "activated" by conversion to its sodium alkoxide by treatment with sodium hydride (NaH) in anhydrous tetrahydrofuran (THF) at about 20 °C under an inert atmosphere which, when reacted

with a triflate [diethyl-(trifluoromethylsulfonyloxymethane) phosphonate] (i.e. $TfOCH_2P(O)(OEt)_2$) in the presence of the catalyst 18 Crown-6 at -15° to 0°C in tetrahydrofuran forms the cyclopentoxymethyl phosphonic acid diesters derivatives of formula 5. In the instance wherein compounds 5 bear a $Z_2$ substituent (i.e. Z is H or F), the compounds may be de-esterified by reaction with trimethylsilylbromide (TMSBr) in anhydrous $CH_2Cl_2$ at about 0°C under an inert atmosphere. The de-esterified compounds may then be subjected to reactions to modify the 6- and/or 2-position substituents of the purine base. Alternatively, compounds 5b (bearing the $Z_3$ substituent) may be converted to their corresponding azides by treatment with tetrabutyl ammonium fluoride ($Bu_4NF$) in tetrahydrofuran to yield a hydroxy derivative which upon treatment with a triflic anhydride ($Tf_2O$) (i.e. $F_3CS(O)_2O\text{-}S(O)_2CF_3$) in pyridine yields a triflate deri-vative which in turn is converted to its epimeric alcohol by reaction with sodium nitrite ($NaNO_2$). The resulting alcohol is first transformed into a triflate by treatment with triflic anhydride/pyridine and is then subjected to a nucleophilic substitution using sodium azide ($NaN_3$) in dimethylformamide to prepare the desired azide analogs.

The diethyl phosphonic acid esters are de-esterified by treatment with TMS Br prior to modifications on the purine base. To prepare 6-amino or 2,6-diamino (or their derivatives) the corresponding 6-chloro or 2,6-chloro purine analogs are reacted with the appropriates amines (i.e. $NH_3$ or $NH_2R$) in methanol under pressure at about 100°C. In those instances wherein the X substituent is OR the corresponding 6-chloro purines are reacted with an acid (HCl/HOH) to obtain the corresponding 6-OH analogs, which may be converted to their alkoxy analogues by standard procedures. The 6-chloro compounds may also be treated with methyl mercaptan to obtain the $-SCH_3$ derivatives using standard procedures.

To prepare compounds of the formula

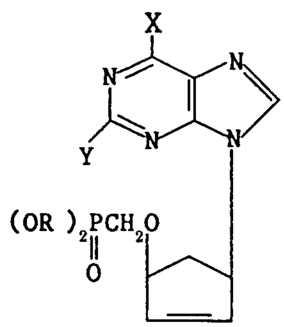

Ic

wherein R , X and Y are as defined in formula I, the processes depicted by the following reaction scheme may be utilized.

<u>REACTION SCHEME B</u>

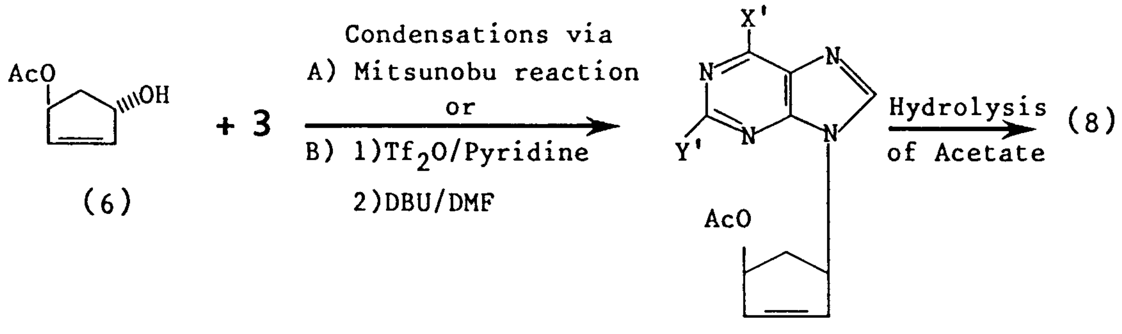


(6) (7)

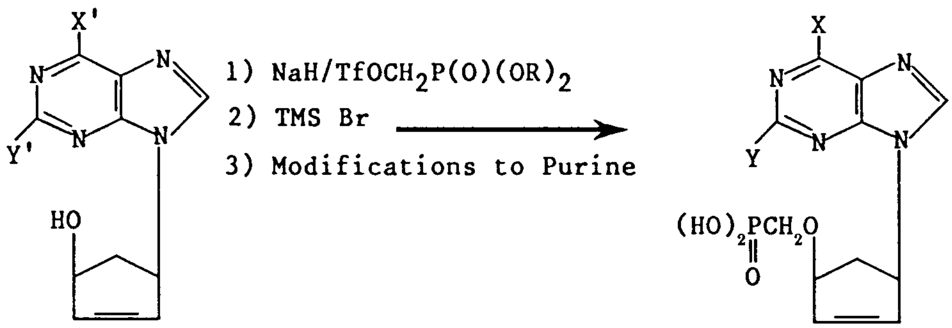


(8) (Id)

wherein Ac is an acyl moiety, preferably

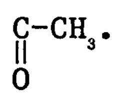

Alternate methods may be employed for the condensation of the purine (3) and the cyclopentene of formula 6. Using the milder conditions of the Mitsunobu-type reaction the condensation takes place in the presence of a phosphine $P(R_3)_3$, $R_3$ being phenyl, methyl or isopropyl using diethyl azodicarboxylate (DEAD) said reaction taking place in a suitable solvent such as tetrahydrofuran at about 0° to 60° C to produce compounds (7). The second method for the condensation of the reactants (3) and (6) entails the activation of the alcohol with triflic anhydride ($TfO_2$) in pyridine using $CH_2Cl_2$ as a solvent and then condensing the activated alcohol with the purine base (3) in the presence of a base e.g. (DBU), KOH, $K_2CO_3$, or other suitable base in dimethylformamide as described above. The acetate of the resulting products (7) is hydrolyzed using catalytic hydrolysis with sodium methoxide in methanol to produce compounds (8) which, as in Reaction Scheme A, are treated with sodium hydride and then with $TfOCH_2P(O)(OR')_2$ in the presence of 18 Crown-6 to produce compounds (1d) which are, again as in Reaction Scheme A (going from 5a to 1a) treated with trimethylsilyl bromide to remove the ester groups of the phosphonate and the resulting products then subjected to the above described purine modifications to obtain the desired X, Y-substituted compounds embraced by formula 1c.

To prepare compounds of this invention having the sub-generic formula

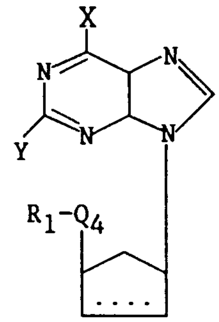

Ie

wherein X, Y and $R_1$ are as previously defined and $Q_4$ is $CH_2OCH_2$- or

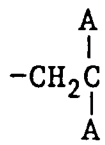

with A being as previously defined (i.e., $R_1Q_4$ is $-CH_2OCH_2P(O)(OR')_2$ or

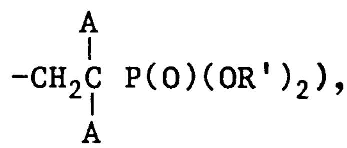

the processes of the following reaction scheme may be utilized.

<u>REACTION SCHEME C</u>

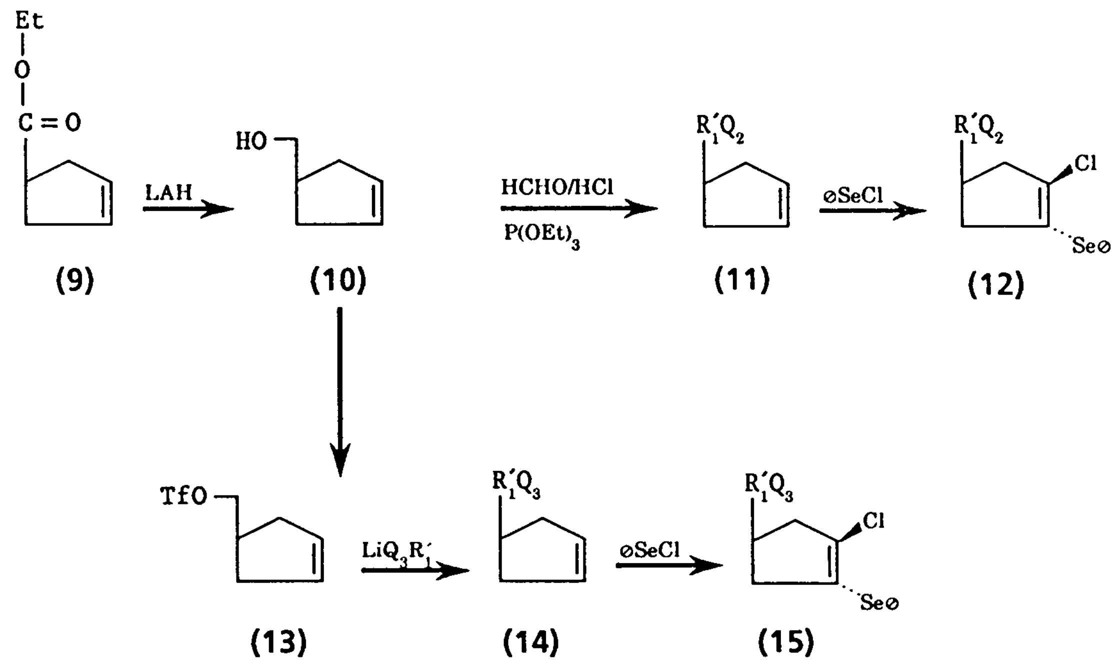


(9) (10) (11) (12)

(13) (14) (15)

(12) and/or (15)

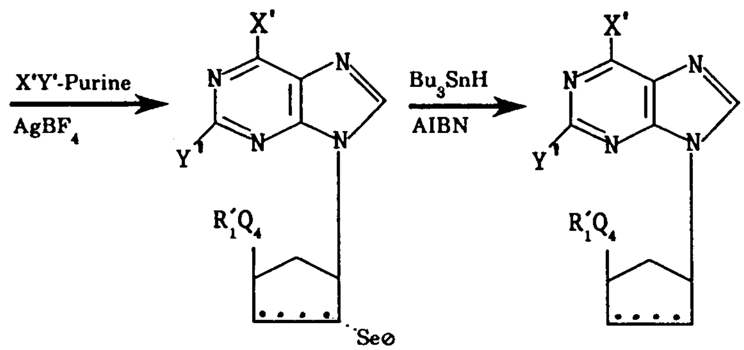


(16) (17)

wherein X', Y', $R_1'Q_4$ are as previously defined, $R_1'Q_3$ is

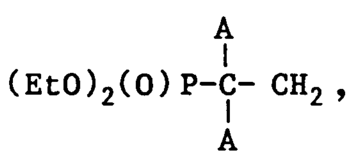

$R_1'Q_2$ is $(EtO)_2(O)PCH_2OCH_2$ and ⌀ is phenyl.

The process is initiated by reducing the ethyl ester of 4-carboxy cyclopentene (9) with lithium aluminum hydride (LAH) to the corresponding hydroxymethyl analog (10). Sequential treatment of (10) with formaldehyde (preferably paraformaldehyde) in the presence of HCl, followed by treatment of the resulting product with an ester of phosphite [preferably $P(OEt)_3$] will yield compounds of formula (II). Alternatively compounds (10) may be activated by reaction with triflic anhydride in the presence of pyridine and the resulting compounds (13) reacted with a lithio derivative of the diethyl ester of the appropriately A,A-disubstituted phosphonic acid, i.e., $Li-C(A)(A)P(O)(OEt)_2$, such as for example the lithio derivative of the diethyl ester of difluoromethyl phosphonic acid [i.e., $Li-CF_2P(O)(OEt)_2$] to produce compounds (14). Following the preparation of compounds (11) and (14), they are treated with phenylselenenyl chloride (⌀ SeCl) in anhydrous dichloromethane under argon at about 20 °C to yield intermediates (12) and (15) which

are condensed with a 6-X',2-Y'-purine in the presence of calcium carbonate and silver tetrafluoroborate ($AgBF_4$) in nitromethane at about 20 °C under argon to produce compounds (16). The phenylselenyl moiety (Se∅) is removed by reaction with tributyltin hhydride ($Bu_3SnH$) in the presence of azoisobutyronitrile (AIBN) to produce compounds (17) which may then be de-esterified and the desired modifications to the purine substitutents effected as previously described following Reaction Scheme A to produce the compounds

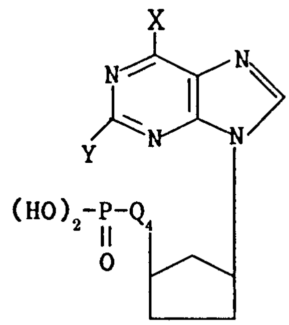

**If**

To prepare compounds of this invention having the sub-generic formula

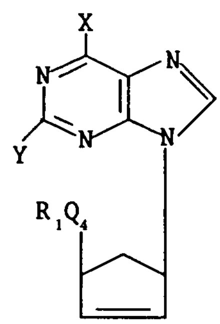

**Ig**

wherein X, Y, $Q_4$ and $R_1$ are as previously defined, the processes illustrated by the following reaction scheme may be utilized.

REACTION SCHEME D

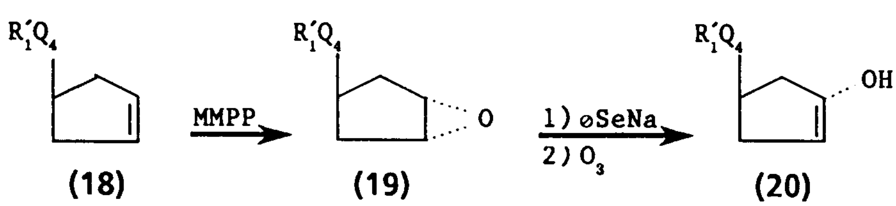


**(18)** **(19)** **(20)**

wherein $R_1'$ and $Q_4$ are as previously defined.

Compounds (18) may be converted to their respective allylic alcohols by formation of an epoxide oxidation with meta-chloroperbenzoic acid (MCPBA) ore preferably, from a safety point of view, with magnesium monoperoxyphtalate (MMPP), followed by an opening of the epoxide (19) by reaction with sodium selenium phenylate followed by treatment with ozone at temperatures of about -78 °C to +20 °C in $CH_2Cl_2$ to produce the desired stereoisomeric form of compounds (20). With less certainty to obtain the desired stereoisomeric form of the desired allylic alcohol (20)n compounds (18) may be subjected to a one-

step oxidation using $^{7}O_2$ oxygen. Once obtained, compounds (20) may be condensed with the X', Y' purine base compounds (3) using either the Mitsunobu or the triflic anhydride series of reactions as previously described, followed by the above-described reactions to modify the X and Y substituents of the purine base.

In the special instance wherein it is desired to prepare compounds of the formula

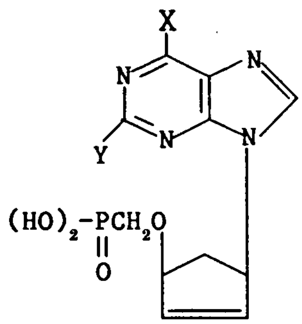

Ih

wherein X and Y are as previously defined, the processes illustrated in the following reaction scheme represent an alternate method for their preparation

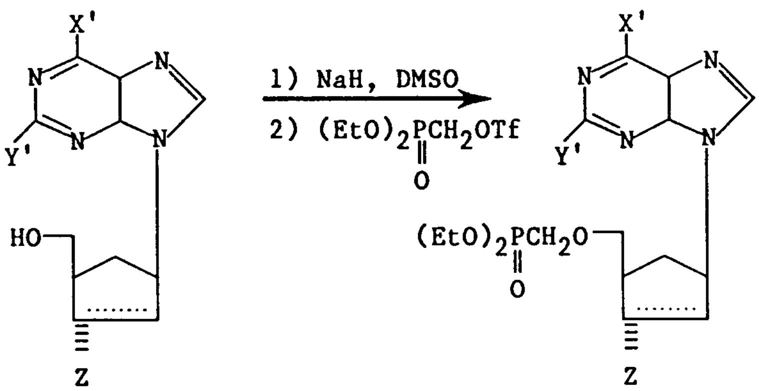


(21) (22)

wherein X' and Y' are as previously defined.

This process involves the activation of the depicted alcohol (2) with sodium hydride in dimethylsulfoxide followed by reaction with the triflate [$(EtO)_2P(O)CH_2OTf$] to produce the compounds of (22) which may be de-esterified (with TMSBr) and subjected to modifications of the purine base as described above.

To prepare compounds of this invention having the sub-generic formula

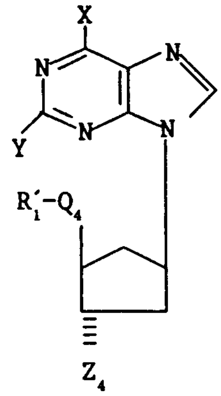

Ij

wherein X and Y are as previously defined and $Z_4$ is $N_3$ or F, the processes illustrated in the following reaction scheme may be utilized.

REACTION SCHEME E

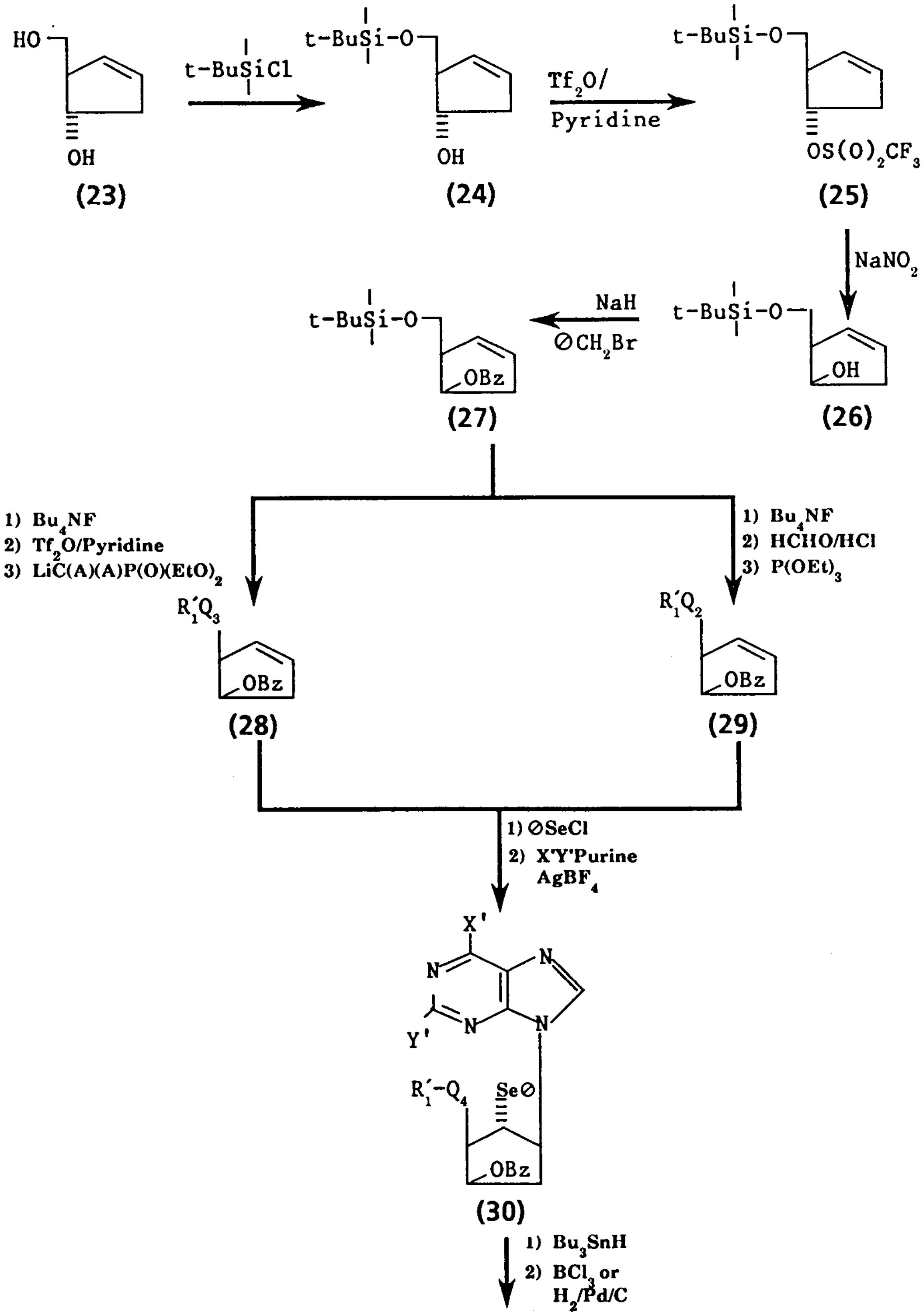


(23) (24) (25) (26) (27) (28) (29) (30)

REACTION SCHEME E (cont'd)

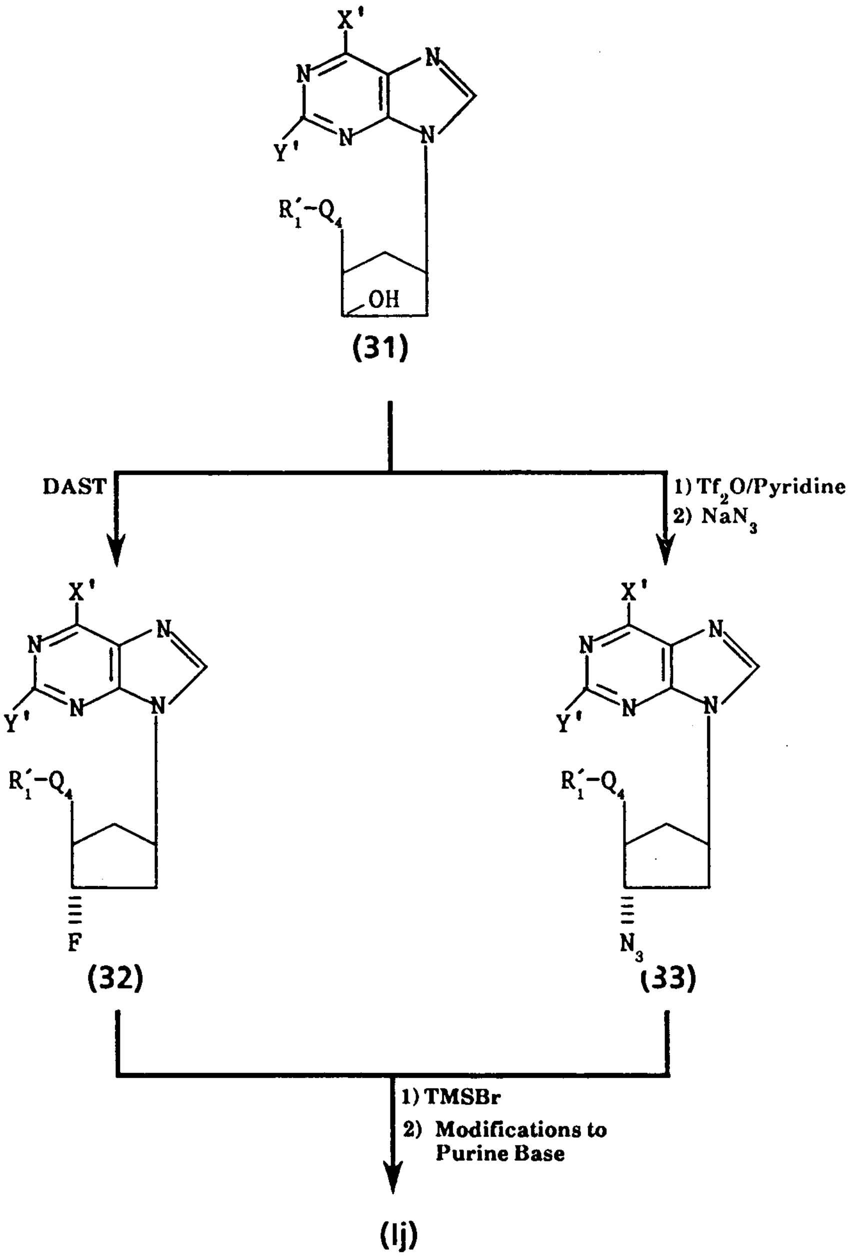


wherein X', Y', X, Y, $R_1'Q_4$, $R_1'Q_2$, and $R_1'Q_3$ are as previously defined,Bz represents benzyl and

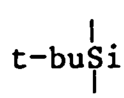

represents t-butyl dimethylsilyl.

The reaction series is initiated by the selective protection of the primary alcohol of compounds (23) by reaction with dimethyl-t-butyl silylchloride to produce compounds (24) which, by sequential treatment with triflic anhydride in the presence of pyridine and then treatment of compounds (25) with sodium nitrite ($NaNO_2$), inverts the alcohol which is then protected with a benzyl protecting group by reaction with benzyl bromide (⌀$CH_2Br$) to yield compounds (27). Following the preparation of compounds (27), the t-butyldimethylsilyl protecting group is removed by treatment with tetrabutylammonium fluoride in tetrahydrofuran and, as in Reaction Scheme C, the so-deprotected analog of compound (27) is alternatively (a) sequentially treated with $Tf_2O$/pyridine and then with $LiC(A)(A)P(O)(EtO)_2$ to produce compounds (28) or (b) sequentially

treated with HCHO/HCl and then $P(OEt)_3$ to produce compounds (29). Again, as analogously described in Reaction Scheme C, compounds (28) and (29) are sequentially reacted with phenylselenium chloride and the products thereof condensed with the X',Y'-purine base to produce compounds (30). Treatment of compounds (30) with tributyltin hydride in the presence of AIBN, as previously described, removes the phenylselenyl (Se⌀) moiety. Hydrolysis of the resulting product with either hydrogen in the presence of palladium on carbon or preferably with boron trichloride removes the benzyl protecting group to produce compounds (31). Again, as analogously described above, treatment of compounds (31) with $Tf_2O$ in the presence of pyridine and then with sodium azide produces the azides of compounds (33).Following preparation of compounds (32) and (33), compounds are de-esterified with TMSBr and followed by the desired modifications to the X,Y-substituents of the purine base. Of course, in those instances wherein it is desired to modify the purine base bearing the alkyl esters, the modifications may be effected without the de-esterification with TMSBr.

The following examples illustrate the preparation of the compounds of this invention.

**EXAMPLE 1**

**Preparation of [3-(6-Amino-9H-purin-9-yl)cyclopentoxymethyl]phosphonic acid**

Step A:
Preparation of 3-(6-chloro-9H-purin-9-yl)cyclopentanol

200 mg of 1,8-diazabicyclo[5.4.0]undec-7-ene (DBU) are added to a stirred suspension of 6-chloropurine (5 g, 32.5 mmol) in 60 ml of anhydrous dimethylformamide containing 7.5 g (91 mmol) of 2-cyclopentenone at 20 °C under argon. The reaction mixture is stirred at 20 °C for 50 hours, and evaporated to dryness under reduced pressure. The solid pale yellow residue is suspended in 150 ml of ethanol at -15 °C and treated with 0.8 g of sodium borohydride which is added portionwise. The reaction mixture is stirred at -15 °C for 3 hours, at 20 °C for 18 hours and 3 ml of acetone are added followed by 0.5 ml of acetic acid. The crude mixture is evaporated under reduced pressure and directly purified by flash chromatography on silica gel using chloroform and increasing amounts of methanol as eluents. The expected product 3-(6-chloro-9H-purin-9-yl)cyclopentanol is obtained as a white solid (3.1 g, 40% yield).

Step B:
Preparation of [3-(6-chloro-9H-purin-9-yl)cyclopentoxymethyl] phosphonic acid, diethyl ester

10 mmol (2.38 g) of 3-(6-chloro-9H-purin-9-yl)-cyclopentanol are added portionwise to a stirred suspension of sodium hydride (10 mmol, 0.4 g, 60% in oil, washed three times with anhydrous hexane) in 25 ml of anhydrous tetrahydrofuran at 0 °C under argon. The reaction mixture is stirred at 20 °C for 20 minutes and 10 mmol (2.65 g) of 18-crown-6 are added to the reaction mixture which is then cooled to -15 °C. 12 mmol of diethyl(trifluoromethylsulfonyloxymethane)phosphonate(3 g) dissolved in 15 ml of tetrahydrofuran are slowly added to the mixture which is stirred at 0 °C for 4 hours, quenched with saturated aqueous ammonium chloride and evaporated. The residue is extracted with ethyl acetate (4 × 70 ml), washed with brine, dried over sodium sulfate, filtered and evaporated to give 6 g of crude product which is purified by flash chromatography on silica gel using chloroform and increasing concentrations of methanol as eluents. 1.75 g of expected product [3-(6-chloro-9H-purin-9-yl)cyclopentoxymethyl] phosphonic acid, diethyl ester is recovered (45% yield).

Step C:
Preparation of [3-(6-chloro-9H-purin-9-yl)cyclopentoxymethyl] phosphonic acid

12 mmol of trimethylsilylbromide (1.85 g) are slowly added to a stirred solution of 1.75 g of [3-(6-chloro-9H-purin-9-yl)cyclopentoxymethyl] phosphonic acid diethyl ester (4.5 mmol) in 15 ml of anhydrous dichloromethane at 0 °C under argon. The reaction mixture is stirred at 20 °C for 20 hours and evaporated under reduced pressure. The residue is dissolved in anhydrous acetonitrile (6 ml) and the product [3-(6-chloro-9H-purin-9-yl)cyclopentoxymethyl] phosphonic acid is precipitated by addition of 200 mg of water. The pale orange solid is collected by filtration (1.35 g) and used in the next step without further purification.

Step D:
Preparation of [3-(6-amino-9H-purin-9-yl)cyclopentoxymethyl]phosphonic acid

4 mmol of [3-(6-chloro-9H-purin-9-yl)cyclopentoxymethyl] phosphonic acid (1.33 g) are added to 100 ml of saturated methanolic ammonia and heated in a steel cylinder at 100 °C for 24 hours. The reaction mixture is cooled at 0 °C and the precipitated solid is recovered and recrystallized from methanol giving 2 mmol of [3-(6-amino-9H-purin-9-yl)cyclopentoxymethyl]phosphonic acid -625 mg, 50% yield).

**EXAMPLE 2**

**Preparation of [2-[3-(6-amino-9H-purin-9-yl)cyclopentyl]-1,1-difluoroethyl]phosphonic acid**

Step A:
Preparation of 3-cyclopentenylmethanol

0.78 mol (109.45 g) of 3-cyclopentenylcarboxylic acid ethyl ester dissolved in 400 ml of anhydrous ether are added dropwise to a stirred suspension of 24 g of lithium aluminum hydride (0.7 mmol) in 500 ml of anhydrous ether. The reaction mixture is stirred at 20 °C for 3 hours and hydrolized by careful addition of 24 ml of water, 24 ml of 15% aqueous sodium hydroxide and three times 24 ml of water. The white precipitate is discarded by filtration and the filtrate is washed twice with brine, dried over sodium sulfate, filtered and evaporated to give 67.5 g of the product 3-cyclopentenylmethanol (88% yield) which is used in the next step without further purification.

Step B:
Synthesis of trifluoromethylsulfonyloxy-3-cyclopentenylmethane

20 ml of triflic anhydride (120 mmol) are added dropwise to a stirred solution of 11 ml of pyridine dissolved in 45 ml of anhydrous dichloromethane at -10 °C under argon. The white suspension is stirred at -10 °C for 30 minutes, cooled at -20 °C and 6 g of 3-cyclopentenylmethanol dissolved in 20 ml of dichloromethane are slowly added to the reaction mixture which is stirred at 0 °C for 4 hours, hydrolyzed with cold water. The organic phase is rapidly washed three times with cold saturated aqueous ammonium chloride and brine, dried over sodium sulfate, filtered and evaporated to give 13.07 g of a pale brown oil which is used in the next step without further purification.

Step C:
Synthesis of 2-[3-cyclopenten-1-yl]1,1-difluoroethylphosphonic acid, diethyl ester

100 mmol (18.8 g) of difluoromethyl phosphonic acid, diethyl ester dissolved in 60 ml of tetrahydrofuran are slowly added to a stirred solution of lithium diisopropylamide (100 mmol) in 60 ml of anhydrous tetrahydrofuran, at -78 °C under argon. The reaction mixture is stirred at -78 °C for 35 minutes and 57 mmol (13.07 g) of the triflate trifluoromethylsulfonyloxy-3-cyclopentenylmethane dissolved in 60 ml of tetrahydrofuran are slowly added at -78 °C. After 6 hours at -78 °C and 2 hours at 20 °C, the reaction mixture is quenched by addition of 50 ml of saturated aqueous ammonium chloride and evaporated under reduced pressure. The residue is dissolved in 300 ml of ethyl acetate, washed with water and brine, dried over sodium sulfate, filtered, evaporated and purified by flash chromatography on silica gel using petroleum ether and increasing amounts of ethyl acetate as eluents giving 7.9 g of the product 2-[3-cyclopenten-1-yl]-1,1-difluoroethylphosphonic acid, diethyl ester, (52% yield).

Step D:
Synthesis of [2-[3-(6-chloro-9H-purin-9-yl)-4-phenylselenylcyclopentyl]-1,1-difluoroethyl]phosphonic acid, diethyl ester

3.56 g (18.7 mmol) of phenylselenenyl chloride are added protionwise to a stirred solution of 5 g (18.7 mmol) of 2-[3-cyclopentenyl]1,1-difluoroethylphosphonic acid, diethyl ester in 30 ml of anhydrous dichloromethane at 20 °C under argon. After 6 hours at 20 °C, the reaction mixture is evaporated under reduced pressure and the residue is dissolved in 30 ml of nitromethane and treated successively by 19 mmol (2.94 g) of 6-chloropurine, 1.8 g of calcium carbonate and 4 g of silver tetrafluoroborate at 20 °C under argon. After 48 hours at 20 °C, the orange-green suspension is evaporated under reduced pressure and directly purified by flash chromatography on silica gel using chloroform and increasing amounts of methanol as eluents giving 3.17 g of the product [2-[3-(6-chloro-9H-purin-9-yl)-4-phenylselenylcyclopentyl]-1,1-difluoroethyl]phosphonic acid, diethyl ester (30% yield).

Step E:
Synthesis of [2-[3-(6-chloro-9H-purin-9-yl)cyclopentyl]-1,1-difluoroethyl]phosphonic acid, diethyl ester

2.64 ml of tributyltinhydride (9.8 mmol) are added to a stirred solution of [2-[3-(6-chloro-9H-purin-9-yl)-4-phenylselenylcyclopentyl]-1,1-difluoroethyl]phosphonic acid, diethyl ester (2.83 g, 4.9 mmol) and 160 mg of azoisobutyronitrile (AIBN) dissolved in 30 ml of toluene. The reaction mixture is stirred at 75-80 °C for 5 hours and at 20 °C for 20 hours, evaporated under reduced pressure and directly purified by flash chromatography on silica gel giving 1.26 g (3 mmol) of [2-[3-(6-chloro-9H-purin-9-yl)cyclopentyl]-1,1-difluoroethyl]phosphonic acid, diethyl ester.

Step F:
Synthesis of [2-[3-(6-amino-9H-purin-9-yl)cyclopentyl]-1,1-difluoroethyl]phosphonic acid

1.6 ml (12 mmol) of trimethylsilylbromide is added to a stirred solution of 1.25 g of [2-[3-(6-chloro-9H-purin-9-yl)cyclopentyl]-1,1-difluoroethyl]phosphonic acid, diethyl ester dissolved in 20 ml of anhydrous dichloromethane at 20 °C under argon. The pale brown solution is evaporated under reduced pressure, the residue is dissolved in anhydrous acetonitrile and an orange solid is precipitated upon addition of 0.25 ml of water. The solid is collected by filtration and suspended in 80 ml of methanol saturated with ammonia in a steel cylinder which is heated at 100 °C for 20 hours. The reaction mixture is cooled at 20 °C and a white solid is collected by filtration. The pure final product [2-[3-(6-amino-9H-purin-9-yl)cyclopentyl]-1,1-difluoroethyl]phosphonic acid is obtained after recrystallization from methanol (430 mg).

**EXAMPLE 3**

**Preparation of {[3-(6-amino-9H-purin-9-yl)cyclopentyl]methoxymethyl}phosphonic acid**

Step A:
Synthesis of 3-[(cyclopenten-1-yl)methoxymethyl]phosphonic acid, diethyl ether

Hydrochloric acid gas is vigorously bubbled into a solution of 10 g of 3-cyclopentenylmethanol and 3 g of paraformaldehyde in 100 ml of anhydrous dichloromethane at 0 °C for 10 minutes. Gentle hydrochloric acid bubbling is continued at 0 °C for 4 hours; excess hydrochloric acid is removed by bubbling nitrogen and the resulting solution is evaporated under reduced pressure giving 13 g of an oil which is added to 15.3 ml of triethylphosphite. The resulting mixture is stirred at 70 °C for 20 hours. The crude product (21 g) is then directly purified by flash chromatography on silica gel using petroleun ether and increasing amounts of ethyl acetate as eluents giving 14.56 g of the product 3-[(cyclopenten-1-yl)methoxymethyl]phosphonic acid, diethyl ether (61% yield).

Step B:
Synthesis of {[3-(6-chloro-9H-purin-9-yl)-4-phenylselenylcyclopentyl]methoxymethyl} phosphonic acid, diethyl ether

2.6 g of {[3-(6-chloro-9H-purin-9-yl)-4-phenylselenylcyclopentyl]methoxymethyl} phosphonic acid, diethyl ether have been prepared from 5 g of 3-[(cyclopenten-1-yl)methoxy methyl]phosphonic acid, diethyl ether, 3.9 g of phenylselenenyl chloride, 4.3 g of silver tetrafluoroborate, 4.6 g of 6-chloropurine and 2 g of calcium carbonate according to the method described in Example 2, Step D.

Step C:
Synthesis of {[3-(6-chloro-9H-purin-9-yl)-cyclopentyl]-methoxymethyl} phosphonic acid, diethyl ether

1.4 g of {[3-(6-chloro-9H-purin-9-yl)-cyclopentyl]-methoxymethyl}phosphonic acid, diethyl ether has been prepared from 2.32 g of {[3-(6-chloro-9H-purin-9-yl)-4-phenylselenylcyclopentyl]methoxymethyl} phosphonic acid, diethyl ether, 2.32 ml of tributyltinhydride and 120 mg of AIBN according to the method described in Example 2, Step D.

Step D:
Synthesis of {[3-(6-amino-9H-purin-9-yl)cyclopentyl]-methoxymethyl}phosphonic acid

600 mg of the final product {[3-(6-amino-9H-purin-9-yl)cyclopentyl]-methoxymethyl}phosphonic acid have been obtained in two steps from {[3-(6-chloro-9H-purin-9-yl)cyclopentyl]-methoxymethyl} phosphonic acid, diethyl ether according to the method described in Example 2, Step F.

## EXAMPLE 4

### Preparation of {2-[4-(2-amino-1,6-dihydro-6-oxo-9H-purin-9-yl)-2-cyclopenten-1-yl]ethyl}phosphonic acid

Step A:
Preparation of [3-cyclopenten-1-yl)ethyl]phosphonic acid, diethyl ester

100 mmol of n-butyllithium (60 ml of a 1.65N solution in hexene) are added to a stirred solution of 100 mmol (15.2 g) of methyl phosphonic acid, diethyl ester dissolved in 60 l of tetrahydrofuran at -78 °C under argon. After stirring at -78 °C for 1 hour, 75 mmol of [(trifluoromethylsulfonyloxy)cyclopenten-4-yl]methane (17.2 g) dissolved in 75 ml of tetrahydrofuran are added dropwise to the reaction mixture which is stirred at -78 °C for 1 hour, at -40 °C for 2 hours and quenched at 0 °C by addition of 100 ml of saturated aqueous ammonium chloride. The crude mixture is evaporated under reduced pressure; the residue is suspended in 350 ml of ethyl acetate, washed with water and brine, dried over sodium sulfate, filtered, evaporated and purified by flash chromatography on silica gel giving 45 mmol (10.44 g) of [3-cyclopenten-1-yl)ethyl]-phosphonic acid, diethyl ester.

Step B:
Preparation of [2-cyclopent[b]oxiran-4-yl)ethyl]phosphonic acid, diethyl ester

20 mmol (9.88 g) of magnesium monoperoxyphtalate dissolved in 40 ml of water are added dropwise to a stirred solution of [3-cyclopenten-1-yl)ethyl]phosphonic acid, diethyl ester (30 mmol, 6.96 g) in 30 ml of isopropanol at 20 °C. The reaction mixture is stirred at 20 °C for 5 hours and treated with 20 ml of an aqueous saturated thiosulfate solution. The crude mixture is evaporated and extracted with three portions of 100 ml ethyl acetate. The organic layers are gathered, washed twice with bicarbonate and brine, dried over sodium sulfate, filtered, evaporated and purified by flash chromatography on silica gel giving 20 mmol of the expected product [2-cyclopent[b]oxiran-4-yl)ethyl]phosphonic acid, diethyl ester (66% yield) as well as 5 mmol of the other isomer.

Step C:
Preparation of [2-(4-hydroxy-2-cyclopenten-1-yl)ethyl]phosphonic acid, diethyl ester

0.86 g of sodium borohydride is added to a stirred solution of diphenyldiselenide (2.8 g, 9 mmol) in 30 ml of ethanol. After stirring at 20 °C for 1 hour, [2-cyclopent[b]oxiran-4-yl)ethyl]phosphonic acid, diethyl ester (15 mmol, 3.72 g) is added to the reaction mixture which is stirred at 20 °C for 20 hours, quenched with acetone (2 ml) and acetic acid (1 ml) and evaporated under reduced pressure to give a crude yellow oil which is dissolved in ethyl acetate (150 ml), washed with bicarbonate and brine, dried over sodium sulfate, filtered and evaporated to give 6.05 g of a crude oil. This oil is dissolved in 25 ml of anhydrous dichloromethane at -78 °C and oxidized by bubbling ozone in the solution until a blue coloration appears. Nitrogen is then bubbled through the solution and 1.2 ml of triethylamine are added at -78 °C. The reaction mixture is slowly heated to 20 °C, stirred for 3 hours, evaporated under reduced pressure and directly purified by flash chromatography on silica gel using ethyl acetate as eluent to give 2.65 g (10 mmol) of [2-(4-hydroxy-2-cyclopenten-1-yl)ethyl]phosphonic acid, diethyl ester (66% yield).

Step D:
Preparation of [2-[4-(2-amino-1,6-dihydro-6-chloro-9H-purin-9-yl)-2-cyclopenten-1-yl]ethyl]phosphonic acid, diethyl ester

3.5 ml of triflic anhydride (20 mmol) are added dropwise to a stirred solution of 1.85 ml of pyridine in 8 ml of anhydrous dichloromethane at -10 °C under argon. After 30 minutes, 2.65 g (10 mmol) of [2-(4-hydroxy-2-cyclopenten-1-yl)ethyl]phosphonic acid, diethyl ester dissolved in 10 ml of dichloromethane are added to the reaction mixture at -20 °C under argon. After 3 hours at -20 °C, the reaction mixture is diluted with cold dichloromethane and rapidly washed with iced-water, cold saturated ammonium chloride and

brine, dried over sodium sulfate, filtered and evaporated to give 4.1 g of crude triflate which is dissolved in 5 ml of dimethylformamide and added to a stirred suspension of 10 mmol (1.69 g) of 2-amino-6-chloropurine and 11 mmol of potassium carbonate in 25 ml of dimethylformamide at 0 °C under argon. The reaction mixture is stirred at 20 °C for 20 hours, evaporated under reduced pressure and directly purified by flash chromatography on silica gel, affording 6 mmol (2.4 g) of [2-[4-(2-amino-1,6-dihydro-6-chloro-9H-purin-9-yl)-2-cyclopenten-1-yl]ethyl]phosphonic acid, diethyl ester (60% yield).

Step E:
Preparation of {2-[4-(2-amino-1,6-dihydro-6-oxo-9H-purin-9-yl)-2-cyclopenten-1-yl]ethyl}phosphonic acid

22 mmol (3. ml) of trimethylsilylbromide are added to a stirred solution of [2-[4-(2-amino-1,6-dihydro-6-chloro-9H-purin-9-yl)-2-cyclopenten-1-yl]ethyl]phosphonic acid, diethyl ester (2.4 g, 6 mmol) in 25 ml of anhydrous dichloromethane at 20 °C under argon. The reaction mixture is stirred at 20 °C for 30 hours and evaporated to dryness. The residue is dissolved in acetonitrile and a pale orange solid is precipitated by addition of water. The solid is collected by filtration, dissolved in 20 ml 1N HCl and 5 ml tetrahydrofuran and heated at 90-95 °C for 18 hours. A white precipitate is formed when the reaction mixture is cooled to 0 °C and the final product {2-[4-(2-amino-1,6-dihydro-6-oxo-9H-purin-9-yl)-2-cyclopenten-1-yl] ethyl} phosphonic acid is isolated after recrystallization from water (0.81 g, 2.15 mmol).

**EXAMPLE 5**

**Preparation of [3-(6-methylamino-9H-purin-9-yl)-cyclopentoxymethyl]phosphonic acid**

A solution of 4 mmol (1.33 g) of [3-(6-chloro-9H-purin-9-yl)cyclopentoxymethyl]phosphonic acid (prepared as described in Example 1, Step C) and methylamine (20 ml of a 40% solution in water) in methanol (100 ml) is heated at 110 °C in a steel bomb for 19 hours. After cooling, the solvents are removed by distillation under vacuum. The residual syrup is dissolved in hot methanol and the final product [3-(6-methylamino-9H-purin-9-yl)-cyclopentoxymethyl]-phosphonic acid (680 mg) is obtained after cristallization on cooling.

**EXAMPLE 6**

**Preparation of {[3-(6-cyclopropylamino-9H-purin-9-yl)-cyclopentyl]-methoxymethyl}phosphonic acid**

12 mmol (1.6 ml) of trimethylsilylbromide are added to a stirred solution of 1.35 g of {[3-(6-chloro-9H-purin-9-yl)-cyclopentyl]methoxymethyl}phosphonic acid, diethyl ester (prepared as described in Example 3, Step C) dissolved in 20 ml of anhydrous dichloromethane at 20 °C under argon. The yellow solution is evaporated under reduced pressure, the residue is dissolved in anhydrous acetonitrile and a pale yellow solid is precipitated upon addition of 0.25 ml of water. The solid is collected by filtration and suspended in 80 ml of methanol containing 5 g of cyclopropylamine in a steel cylinder which is heated at 100 °C for 20 hours. The reaction mixture is cooled at 20 °C and a white solid is collected by filtration. The expected final product {[3-(6-cyclopropylamino-9H-purin-9-yl)-cyclopentyl]methoxymethyl}phosphonic acid is obtained in a pure form after two recrystallizations from hot methanol (380 mg).

**EXAMPLE 7**

**Preparation of {[4-(2-amino-1,6-dihydro-6-oxo-9H-purin-9-yl)-2-cyclopenten-1-yl]-methoxymethyl}phosphonic acid**

Step A:
Preparation of {[4-(2-amino-1,6-dihydro-6-oxo-9H-purin-9-yl)-2-cyclopenten-1-yl]methoxymethyl}phosphonic acid, diethyl ester

10 mmol of carbovir (2.5 g) are added portionwise to a stirred suspension of sodium hydride (30 mmol, 1.2 g, 60% in oil, washed three times with anhydrous hexane) in 35 ml of anhydrous dimethylsulfoxide at 20 °C under argon. The reaction mixture is stirred at 20 °C for 30 minutes and 20 mmol (5.30 g) of diethyl-(trifluoromethylsulfonyloxymethane)phosphonate dissolved in 20 ml of dimethylsulfoxide are slowly added to

the mixture which is stirred at 20°C for 20 hours, quenched with saturated aqueous ammonium chloride and evaporated to dryness under reduced pressure. The residue is suspended in 100 ml of 0.1N HCl solution in water. The aqueous phase is washed 5 times with 35 ml portions of ether, stirred on charcoal, filtered and evaporated. The residue is dissolved in hot water and 1.59 g of the expected product {[4-(2-amino-1,6-dihydro-6-oxo-9H-purin-9-yl)-2-cyclopenten-1-yl]methoxymethyl}phosphonic acid, diethyl ester is obtained by precipitation on cooling (40% yield).

Step B:

Preparation of {[4-(2-amino-1,6-dihydro-6-oxo-9H-purin-9-yl)-2-cyclopenten-1-yl]methoxymethyl}phosphonic acid

18 mmol (2.4 ml) of trimethylsilylbromide are added to a stirred solution of 1.59 g (4 mmol) of {[4-(2-amino-1,6-dihydro-6-oxo-9H-purin-9-yl)-2-cyclopenten-1-yl]methoxymethyl}phosphonic acid, diethylester in 25 ml of anhydrous dimethylformamide at 20°C under argon. The reaction mixture is stirred at 20°C for 36 hours and evaporated to dryness. The crude residue is dissolved in 30 ml of acetonitrile and the final product is obtained as a white solid on addition of 0.5 ml of water. Further recrystallization from hot water gives 800 mg of the expect product {[4-(2-amino-1,6-dihydro-6-oxo-9H-purin-9-yl)-2-cyclopenten-1-yl]-methoxymethyl}phosphonic acid.

The compounds of this invention are useful in the medical therapy particularly for the treatment and prophylaxis of retroviral infections and hepatitis B viral infections.

Examples of retroviral infections which may be treated or prevented in accordance with the invention include human retroviral infections such as Human Immunodeficiency Virus (HIV), HIV-2 and Human T-cell Lymphotropic Virus (HLTV) e.g. HTLV-I or HTLV-IV infections. The compounds according to the invention are especially useful for the treatment or prophylaxis of AIDS and related clinical conditions such as AIDS-related complex (ARC), progressive generalized lymphadenopathy (PGL), AIDS-related neurological conditions, such as multiple sclerosis or tropical paraparesis, anti-HIV antibody-positive and HIV-positive conditions, Kaposi's sarcoma and thrombocytopenic purpura. The compounds may also be used in the treatment or prevention of psoriasis.

In a further aspect of the present invention there is included: a) a method for the treatment and prophylaxis of retroviral infections and hepatitis B infections which comprises treating the subject with a therapeutically effective amount of a compound according to the invention; b) use of a compound according to the invention in the manufacture of a medicament for the treatment or prophylaxis of any of the above-mentioned infections or conditions.

The compounds of this invention may be employed in combination with other therapeutic agents for the treatment or prophylaxis of the above infections or conditions. Examples of such further therapeutic agents include agents that are effective for the treatment or prophylaxis of viral infections or associated conditions such as 3'-azido-3'-deoxythymidine (zidovudine), 2',3'-dideoxynucleosides such as 2',3'-dideoxycytidine, 2',3'-dideoxy adenosine and 2',3'-dideoxyinosine, acyclic nucleosides (e.g. acyclovir), interferons such as $\alpha$-interferon, renal excretion inhibitors such as probenicid, nucleoside transport inhibitors such as dipyridamole, as well as immunomodulators such as interleukin II and granulocyte macrophage colony stimulating factors. The component compounds of such combination therapy may be administered simultaneously, in either separate or combined formulations, or at different times, e.g. sequentially such that a combined effect is achieved.

The compounds according to the invention, also referred to herein as the active ingredient, may be administered for therapy by any suitable route including oral, rectal, nasal, topical (including buccal and sublingual), vaginal and parenteral (including subcutaneous, intramuscular, intravenous and intradermal). It will be appreciated that the preferred route will vary with the condition and age of the recipient, the nature of the infection and the chosen active ingredient.

In general a suitable dose will be in the range of 3.0 to 120 mg per kilogram body weight of the recipient per day, preferably in the range of 6 to 90 mg per kilogram body weight per day and most preferably in the range of 15 to 60 mg per kilogram body weight per day. The desired dose is preferably presented as two, three, four, five, six or more sub-doses administered at appropriate intervals throughout the day. These sub-doses may be administered in unit dosage forms, for example, containing 10 to 1,500 mg, preferably 20 to 1,000 mg, and most preferably 50 to 700 mg of active ingredient per unit dosage form.

Ideally, the active ingredient should be administered to achieve peak plasma concentrations of the active compound of from about 1 to about 75$\mu$M, preferably about 2 to 50$\mu$M, most preferably about 3 to about 30$\mu$M. This may be achieved, for example, by the intravenous injection of a 0.1 to 5% solution of the active ingredient, optionally in saline, or orally administered as a bolus containing about 1 to about 100

mg/kg of the active ingredient. Desirable blood levels may be maintained by a continuous infusion to provide about 0.01 to about 5.0 mg/kg/hour or by intermittent infusions containing about 0.4 to about 15 mg/kg of the active ingredient.

While it is possible for the active ingredient to be administered alone it is preferable to present it as a pharmaceutical formulation. The formulations of the present invention comprise at least one active ingredient, as defined above, together with one or more acceptable carriers thereof and optionally other therapeutic agents. Each carrier must be "acceptable" in the sense of being compatible with the other ingredients of the formulation and not injurious to the patient. Formulations include those suitable for oral, rectal, nasal, topical (including buccal and sublingual), vaginal or parenteral (including subcutaneous, intramuscular, intravenous and intradermal) administration. The formulations may conveniently be presented in unit dosage form and may be prepared by any methods well known in the art of pharmacy. Such methods include the step of bringing into association the active ingredients with the carrier which constitutes one or more accessory ingredients. In general, the formulations are prepared by uniformly and intimately bringing into association the active ingredient with liquid-carriers or finely divided solid carriers or both, and then if necessary shaping the product.

Formulations of the present invention suitable for oral administration may be presented as discrete units such as capsules, cachets or tablets each containing a predetermined amount of the active ingredient; as a powder or granules; as a solution or a suspension in an aqueous or non-aqueous liquid; or as an oil-in-water liquid emulsion or a water-in-oil liquid emulsion. The active ingredient may also be presented as a bolus, electuary or paste. A tablet may be made by compression or moulding, optionally with one or more accessory ingredients. Compressed tablets may be prepared by compressing in a suitable machine the active ingredient in a free-flowing form such as a powder or granules, optionally mixed with a binder (e.g. povidone, gelatin, hydroxypropylmethyl cellulose), lubricant, inert diluent, preservative, disintegrant (e.g. sodium starch glycollate, cross-linked povidone, cross-linked sodium carboxylmethyl cellulose) surface-active or dispersing agent. Moulded tablets may be made by moulding in a suitable machine a mixture of the powdered compound moistened with an inert liquid diluent. The tablets may optionally be coated or scored and may be formulated so as to provide slow or controlled release of the active ingredient therein using, for example, hydroxypropylmethyl cellulose in varying proportions to provide the desired release profile. Tablets may optionally be provided with an enteric coating, to provide release in parts of the gut other than the stomach. This is particularly advantageous for purine nucleoside derivatives as such compounds are susceptible to acid hydrolysis.

Formulations suitable for topical administration in the mouth include lozenges comprising the active ingredient in a flavored basis, usually sucrose and acacia or tragacanth; pastilles comprising the active ingredient in an inert basis such as gelatin and glycerin, or sucrose and acacia; and mouthwashes comprising the active ingredient in a suitable liquid carrier.

Formulations for rectal administration may be presented as a suppository with a suitable base comprising, for example, cocoa butter or a salicylate.

Formulations suitable for vaginal administration may be presented as pessaries, tampons, creams, gels, pastes, foams or spray formulations containing in addition to the active ingredient such carriers as are known in the art to be appropriate.

Formulations suitable for parenteral administration include aqueous and non-aqueous isotonic sterile injection solutions which may contain anti-oxydants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose sealed containers, for example, ampoules and vials, and may be stored in a freeze-dried (lyophilized) condition requiring only the addition of the sterile liquid carrier, for example water for injections, immediately prior to use. Extemporaneous injection solutions and suspensions may be prepared from sterile powders, granules and tablets of the kind previously described.

Preferred unit dosage formulations are those containing a daily dose or unit, daily sub-dose, as herein above recited, or an appropriate fraction thereof, of an active ingredient.

As is true for many large classes of compounds suitable for use as therapeutic agents certain subclasses and certain specific members will be found to be more effective than others. In this instance those compounds wherein X is OH or $NH_2$ are preferred, those wherein X is $NH_2$ or H are preferred. It is preferred that Z be H or $N_3$ and it is preferred that Q represent either an $-OCH_2$ or a $CH_2OCH_2$ moiety and, of course, it is preferred that the $(OR)_2$ of $R_1$ represent $(OH)_2$.

**Claims**

**1.** A compound of the formula

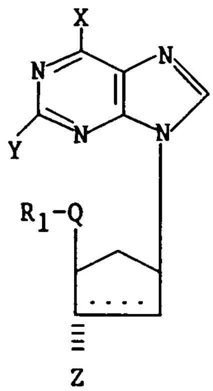

the optical and geometric isomers and mixtures thereof, and the pharmaceutically acceptable salts thereof, wherein the dotted line represents a facultative double bond,

- X is $OR_2$, NHR, Cl, or $SCH_3$ with $R_2$ being a $C_{1-6}$ lower alkyl, H, or $C_{3-6}$ cycloalkyl,
- Y is H or $NH_2$,
- Z is H, F or $N_3$, with the proviso that when the dotted line represents a double bond Z can only be H,
- $R_1$-Q is-$OCH_2P(O)(OR)_2$,-$CH_2OCH_2P(O)(OR)_2$ or -$CH_2C(A)(A)P(O)(OR)_2$, each A independently be H, F or Cl, and each R independently be H or $C_{1-6}$ alkyl.

European
Patent Office

# EUROPEAN SEARCH REPORT

Application Number

**EP 90 40 2129**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 369 409 (BRISTOL-MYERS SQUIBB CO.)<br>* The whole document *<br>– – – – – | 1 | C 07 F 9/6561<br>A 61 K 31/675 |
| | | | **TECHNICAL FIELDS SEARCHED (Int. Cl.5)** |
| | | | C 07 F 9/00<br>A 61 K 31/00 |

The present search report has been drawn up for all claims

| Place of search | Date of completion of search | Examiner |
|---|---|---|
| The Hague | 12 March 91 | BESLIER L.M. |

CATEGORY OF CITED DOCUMENTS

- X : particularly relevant if taken alone
- Y : particularly relevant if combined with another document of the same catagory
- A : technological background
- O : non-written disclosure
- P : intermediate document
- T : theory or principle underlying the invention
- E : earlier patent document, but published on, or after the filing date
- D : document cited in the application
- L : document cited for other reasons
- & : member of the same patent family, corresponding document